# DEMANDE DE BREVET EUROPEEN

(11) **EP 4 474 005 A1**
(43) Date de publication de la demande: **11.12.2024**
(21) Numéro de dépôt: 24180049.9
(22) Date de dépôt: 04.06.2024
(51) Int. Cl.: A61N 5/06

(54) **DISPOSITIF DE TRAITEMENT PAR PHOTOTHÉRAPIE**

(30) Priorité: 07.06.2023 FR 2305753
(71) Demandeur: Lucibel SA, 76360 Barentin (FR)
(72) Inventeur: DAVID, Thibault, 76240 Bonsecours (FR); GONTIER, Paul, 76100 Rouen (FR)
(74) Mandataire: Martin, Marie-Aude

(57) **Abrégé**

Le casque (10) de traitement du cuir chevelu d'un utilisateur par photothérapie, le casque (10) comprenant des parois de coque extérieure et intérieure s'étendant chacune d'une portion de sommet proximale à un bord distal et définissant une fois assemblées, une bordure périphérique du casque qui délimite une ouverture de passage de la tête de l'utilisateur, et une source de lumière (30) disposée entre les deux parois émettant vers l'intérieur du casque (10), la source de lumière (30) comprenant une pluralité de diodes électroluminescentes (34) et un support (32) d'interconnexion électrique des diodes entre elles. Le support (32) est configuré en étoile et comprend une pluralité de branches principales qui s'étendent radialement depuis une région centrale de convergence des branches principales vers une extrémité libre des branches (320).

## Description

La présente invention concerne un dispositif de traitement par photothérapie pour une région externe du corps d'un être vivant. Plus particulièrement mais non exclusivement, elle s'applique au traitement par photothérapie de la surface de tête d'un utilisateur, par exemple au traitement du cuir chevelu en tant que traitement de la calvitie.

De façon connue en soi, le traitement consiste en l'application d'un rayonnement lumineux présentant un spectre d'émission prédéfini directement sur le cuir chevelu. Ce traitement par photothérapie peut être appliqué également en combinaison avec un produit photo-sensibilisant appliqué sur la région à traiter quelques heures avant son exposition au rayonnement lumineux pour augmenter l'efficacité du traitement. On parle alors dans ce cas de photothérapie dynamique (connue également sous l'acronyme anglais PDT « Photo Dynamic Therapy »).

Le rayonnement est de préférence sensiblement monochromatique ou présente un spectre d'émission compris dans une bande relativement étroite de longueurs d'onde. Généralement, le spectre d'émission comprend une longueur d'onde maximale située dans la lumière visible rouge ou encore dans la lumière visible bleue, ces longueurs d'onde ayant démontré une efficacité particulière pour le traitement de certaines affections de la peau et pour le traitement du cuir chevelu.

Il a été ainsi observé au cours de nombreuses études scientifiques, qu'un traitement par photothérapie dans une plage de longueurs d'onde prédéfinie, notamment dans la lumière rouge ou bleue, permet d'agir de façon bénéfique sur de nombreux problèmes cutanés, tels que les cicatrices, les vergetures, l'acné, les rides et ridules et des problèmes capillaires tels que l'alopécie.

Plus particulièrement, lors de l'application du rayonnement sur la peau, notamment dans la lumière rouge et plus particulièrement autour de la longueur d'onde 630 nm, les cellules de la région irradiées vont réagir selon un principe similaire à celui de la photosynthèse des végétaux. La lumière va stimuler les cellules, et plus spécifiquement certaines structures cellulaires de la cellule, telles que les mitochondries.

De façon connue en soi, les mitochondries jouent un rôle particulièrement important dans le processus de respiration cellulaire ainsi que dans le processus de transformation des nutriments en un vecteur énergétique désigné couramment par ATP (acronyme pour Adénosine Tri Phosphate). Dès lors, la stimulation des mitochondries par la lumière rouge contribue à une régénération des cellules grâce à l'amélioration du métabolisme cellulaire. Une telle régénération cellulaire va se manifester par exemple pour les cellules de la peau du visage par une augmentation de la production de collagène et d'élastine et par conséquent une diminution observable des rides et ridules.

Plus particulièrement, il a été observé que l'exposition du cuir chevelu à la lumière rouge, par exemple à la longueur d'onde de 630 nm, permet une stimulation de la repousse capillaire, une amélioration de la qualité et de la densité des cheveux ainsi qu'une stabilisation de la perte de cheveux.

On connaît déjà dans l'état de la technique, en particulier de la demande de brevet EP2477697 un équipement de traitement par photothérapie du cuir chevelu d'un utilisateur, comprenant un casque délimitant une extrémité ouverte par laquelle, en position d'utilisation, la tête de l'utilisateur est au moins partiellement engagée.

L'alopécie se traduit généralement par une perte de cheveux qui est localisée dans une partie sommitale du crâne appelée tonsure ou qui est localisée dans les golfes temporaux et frontaux. Dans l'alopécie masculine, l'éclaircissement progressif des cheveux dans ces deux zones peut alors confluer pour former une couronne basse de cheveux sur les tempes et la nuque.

Bien que l'alopécie masculine soit la plus répandue, la population féminine est également touchée par la perte de cheveux qui se caractérise généralement dans ce cas par un éclaircissement des cheveux le long d'une ligne sommitale.

Il est ainsi bien connu que l'alopécie est une cause de préoccupation importante de la population en général aussi bien pour des raisons esthétiques que pour des raisons psychologiques.

### Description de l'invention

La présente invention a notamment pour but de proposer un dispositif de traitement de photothérapie qui améliore le traitement de l'alopécie masculine et féminine.

A cet effet l'invention a pour objet un casque de traitement du cuir chevelu d'un utilisateur par photothérapie, le casque comprenant des parois de coque extérieure et intérieure s'étendant chacune d'une portion de sommet proximale à un bord distal et définissant une fois assemblées, une bordure périphérique du casque qui délimite une ouverture de passage de la tête de l'utilisateur, et une source de lumière disposée entre les deux parois émettant vers l'intérieur du casque, la source de lumière comprenant une pluralité d'émetteurs lumineux et un support d'interconnexion électrique des émetteurs entre eux, caractérisé en ce que le support est configuré en étoile et comprend une pluralité de branches principales qui s'étendent radialement depuis une région centrale de convergence des branches principales vers une extrémité libre des branches.

Un casque selon l'invention peut comprendre l'une ou plusieurs des caractéristiques suivantes.

Dans un mode de réalisation préféré de l'invention, la densité de puissance est maximale dans la région centrale, par exemple 20% supérieure à la densité de puissance dans la région périphérique s'étendant autour de la région centrale.

Dans un mode de réalisation préféré de l'invention, au moins une branche principale comprend une ramification en une pluralité de branches secondaires partant d'au moins un des bords longitudinaux des branches principales.

Dans un mode de réalisation préféré de l'invention, la ramification des branches diffère d'une branche principale à l'autre.

Dans un mode de réalisation préféré de l'invention, le support est symétrique par rapport à un axe s'étendant depuis une région frontale vers une région occipitale du casque.

Dans un mode de réalisation préféré de l'invention, les émetteurs lumineux sont plus concentrés dans la région centrale et plus dispersés dans la région périphérique.

Dans un mode de réalisation préféré de l'invention, le dispositif comprend en outre une interface de dissipation thermique métallique interposée entre les parois extérieure et intérieure et supportant la source de lumière du côté de sa face interne tournée vers la coque intérieure.

Dans un mode de réalisation préféré de l'invention, l'interface de dissipation thermique est formée par une plaque métallique conformée pour épouser la forme hémisphérique de la cavité du corps de casque.

Dans un mode de réalisation préféré de l'invention, la plaque est obtenue par pliage d'un flan métallique prédécoupé ou entaillé selon des lignes de pliage.

Dans un mode de réalisation préféré de l'invention, la région centrale du support s'étend au droit d'une région sommitale du casque.

Dans un mode de réalisation préféré de l'invention, la région centrale du support s'étend au droit d'une partie arrière ou d'une partie avant de la région sommitale du casque.

Dans un mode de réalisation préféré de l'invention, le dispositif comprend un bandeau circonférentiel d'ajustement du casque à un tour de tête de l'utilisateur, accroché à la bordure périphérique, ledit bandeau comprenant un corps pourvu d'une portion de déformation s'étendant radialement vers l'intérieur du casque et étant configurée pour se déformer entre une position de repos déployée radialement vers l'intérieur du casque et une position tassée entre le casque et la tête de l'utilisateur en configuration d'utilisation.

L'invention sera bien comprise et ses avantages apparaîtront mieux, à la lecture de la description détaillée qui suit, de modes de réalisation représentés à titre d'exemples non limitatifs. La description se réfère aux dessins annexés sur lesquels :
[Fig.1] La [Fig.1] est une vue en perspective d'un casque selon l'invention ;
[Fig.2] La [Fig.2] est une vue de dessous du casque de la [Fig.1] ;
[Fig.3] La [Fig.3] est une vue en coupe du casque de la [Fig.1] :
[Fig.4] La [Fig.4] est une vue en perspective et de dessus d'une couche de dissipation thermique supportant la source lumineuse du casque de la [Fig.1] ;
[Fig.5] La [Fig.5] est une vue en perspective de la source lumineuse comprenant un support et des diodes électroluminescentes de la [Fig.4], dans une configuration mise en forme ;
[Fig.6] La [Fig.6] est une vue de face de la source lumineuse dans une configuration à plat.

### Description détaillée de l'invention

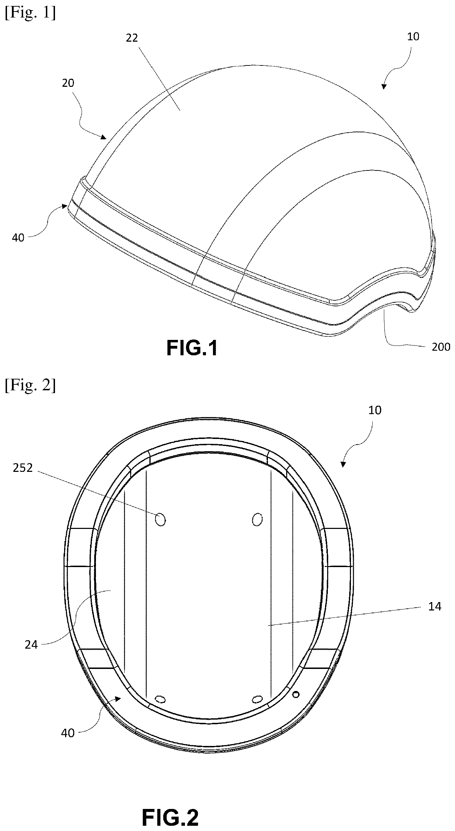

On a représenté sur les figures 1 à 6 un casque de traitement du cuir chevelu selon l'invention. Le casque de traitement est désigné par la référence générale 10.

Comme illustré sur les figures 1 à 3, le casque de traitement 10 comprend un corps 20 qui délimite une cavité 14 à l'intérieur de laquelle un utilisateur peut insérer sa tête.

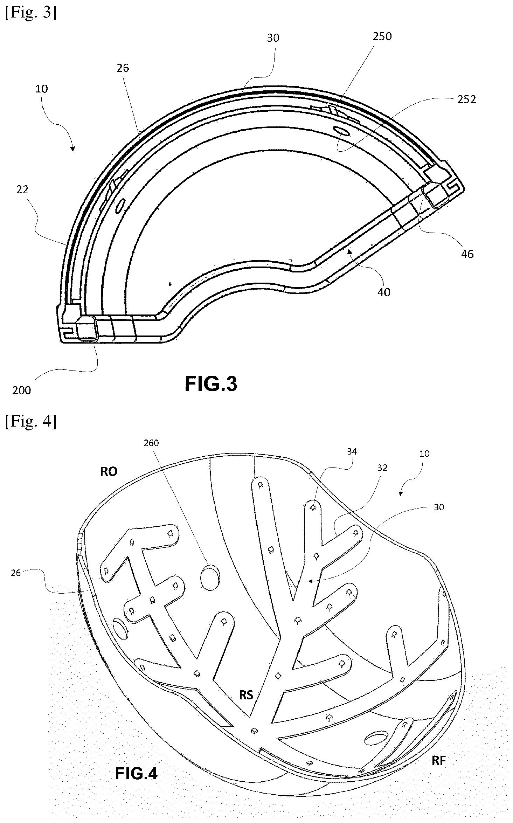

Le corps 20 incorpore encore une source de lumière 30 qui émet vers l'intérieur de la cavité 14 en direction de la surface à traiter. La source de lumière 30 est visible sur la [Fig.4] et comprend dans cet exemple une pluralité de diodes électroluminescentes 34 formant une pluralité d'émetteurs lumineux. Dans l'exemple décrit, les diodes électroluminescentes 34 émettent une lumière rouge de préférence avec une longueur d'onde autour de 630 nanomètres, dans une plage de 620 à 640 nanomètres. En variante, d'autres longueurs d'onde de lumière peuvent être sélectionnées en fonction de la nature du traitement de photothérapie souhaité.

Comme cela est illustré sur les figures, le corps 20 du casque 10 comprend des parois de coque extérieure 22 et intérieure 24 s'étendant chacune d'une portion de sommet proximale jusqu'à un bord distal de la paroi de coque 22, 24. Les deux parois de coque 22, 24, définissent une fois assemblées, une bordure périphérique du casque 10 qui délimite l'ouverture de passage de la tête.

La paroi de coque intérieure 24 est réalisée dans une matière laissant passer le flux lumineux émis par la source de lumière 30. De préférence, la matière est translucide pour permettre une diffusion homogène de la lumière ainsi qu'une protection naturelle de l'utilisateur contre l'éblouissement.

Par translucide, on entend un matériau qui laisse passer la lumière mais qui n'est pas nécessairement transparent. Le choix d'un matériau translucide plutôt qu'un matériau transparent permet également au niveau esthétique de rendre moins visibles les sources lumineuses. Bien entendu, en variante, le matériau peut être choisi transparent aux rayons lumineux émis par les sources lumineuses.

Par exemple, de préférence, la paroi intérieure 24 est réalisée dans une matière plastique thermoformable, par exemple un mélange de polystyrène et de polyéthylène (acronyme PS-PE).

Dans le mode de réalisation préféré de l'invention, la source de lumière 30 est disposée entre les deux parois de coque 22, 24 et émet vers l'intérieur du casque 10.

En outre, de préférence, les deux parois 22, 24 sont accouplées entre elles par des attaches 250, par exemple en matière plastique se présentant sous forme de clips présentant des orifices 252 coïncidant des deux parois 22, 24 afin de recevoir un élément de fixation par vissage par exemple.

Dans le mode de réalisation illustré sur les figures, le casque 10 comprend encore un bandeau circonférentiel 40 d'ajustement du casque 10 à un tour de tête de l'utilisateur, accroché à la bordure périphérique. Ce bandeau 40 permet d'ajuster la cavité du casque 10 au tour de tête de l'utilisateur, ce tour de tête présentant une grande variabilité au sein de la population générale.

En particulier, la source de lumière 30 comprend une pluralité de diodes 34 électroluminescentes et un support 32 d'interconnexion électrique des diodes 34 entre elles.

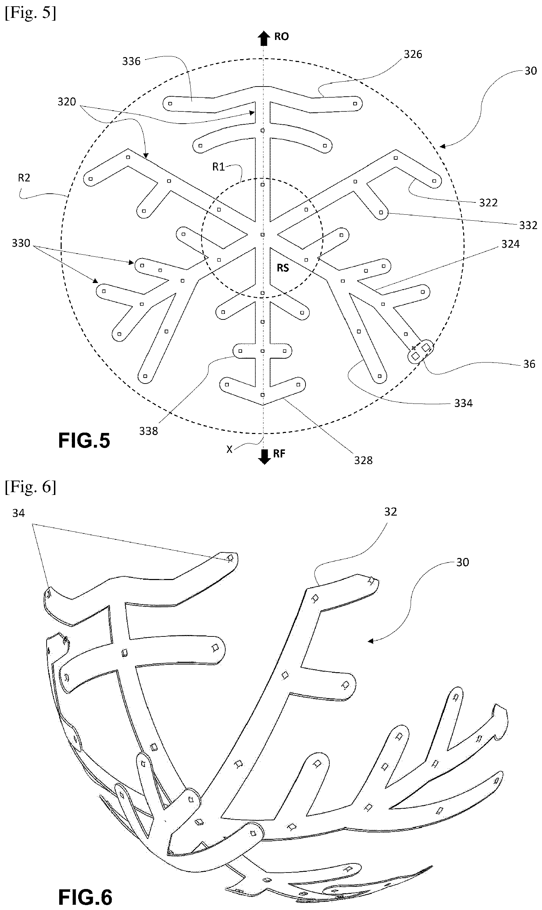

Dans le mode de réalisation préféré de l'invention et tel qu'illustré sur la [Fig.5], le support d'interconnexion électrique 32 est formé à partir d'une plaque ajourée qui est configurée en étoile et qui comprend une pluralité de branches principales 320 qui s'étendent radialement depuis une région centrale R1 de convergence des branches principales 320 vers une extrémité libre des branches, à l'intérieur d'une région périphérique R2. Ce support 26 est également percée par des orifices 260 coïncidant avec les orifices 252 des parois 22, 24,

De préférence, les branches principales 320 sont globalement rectilignes selon la direction radiale avec en extrémité une ligne brisée ou bifurquée.

En outre, comme cela est illustré de façon non limitative sur les figures, au moins une des branches principales 320 comprend à son extrémité libre un bornier 36 de raccordement électrique du support 32 à une source d'alimentation électrique externe au casque 10 afin d'alimenter électriquement la source lumineuse 30.

Dans un mode de réalisation préféré de l'invention, au moins une branche principale 320 comprend une ramification en une pluralité de branches secondaires 330 partant d'au moins un des bords longitudinaux des branches principales 320.

De préférence, la ramification des branches secondaires 330 et/ou bifurcation d'une branche principale 320 diffère d'une branche principale à l'autre 320. Ceci permet d'optimiser la répartition de la densité de puissance de la source lumineuse 30 en fonction de la région du casque 10 et en conséquence des zones du cuir chevelu.

De préférence, le support 32 est symétrique par rapport à un axe X s'étendant depuis une région frontale RF vers une région occipitale RO du casque 10.

Comme illustré sur la [Fig.5] et de préférence, dans la configuration mise en forme dans la cavité du casque 10 ([Fig.6]), le support 32 comprend deux branches principales 324 latérales antérieures qui s'étendent selon les régions des golfes temporaux. Le support 32 comprend encore de préférence deux branches principales 322 latérales postérieures qui sont destinées à s'étendre dans la région supérieure de la couronne occipitale de la tête de l'utilisateur. En outre, de préférence, le support 32 comprend encore une branche principale postérieure centrale 326 destinée à s'étendre dans la région de la couronne occipitale de façon centrale et une branche principale antérieure centrale 328 destinée à s'étendre sur l'avant du casque 10. Dans l'exemple illustré sur la [Fig.5], chacune des branches principales 322, 324, 326 et 328 possèdent des ramifications secondaires 330 correspondantes référencées respectivement 332, 334, 336 et 338.

De préférence, la densité de puissance dans la région centrale R1 est supérieure à la densité de puissance dans la région périphérique R2 s'étendant autour de la région centrale R1, par exemple 20% supérieure. De préférence, la concentration des diodes électroluminescentes 34 est plus importante dans la région centrale R1 que dans la région périphérique R2. Par exemple, les diodes électroluminescentes 34 présentent une concentration plus importante dans la région centrale R1, que dans la région périphérique R2.

Dans un mode de réalisation préféré, le casque 10 comprend en outre une interface ou couche 26 de dissipation thermique, par exemple métallique, interposée entre les parois de coque extérieure 22 et intérieure 24 et supportant la source de lumière 30 du côté de sa face interne tournée vers la coque intérieure 24.

Par exemple, l'interface de dissipation thermique 26 est formée par une plaque métallique, de préférence en aluminium, conformée pour épouser la forme hémisphérique de la cavité du corps 20 de casque 10. De préférence, la plaque 26 est obtenue par pliage d'un flan métallique prédécoupé ou entaillé selon des lignes de pliage préférentielles.

De préférence, la région centrale R1 de forte densité de puissance du support s'étend au droit d'une région sommitale RS du casque 10. Par exemple, la région centrale R1 du support 32 s'étend au droit d'une partie antérieure ou postérieure d'une partie avant de la région sommitale RS du casque 10.

On va maintenant décrire les principaux aspects de fonctionnement d'un casque de photothérapie selon l'invention.

Un utilisateur positionne le casque 10 sur son cuir chevelu. Dans la position d'utilisation, la région sommitale du crâne de l'utilisateur se situe sous la région centrale R1 de la source de lumière 30. Dans cette région centrale R1, la densité de puissance est maximale. Ceci permet d'avoir un traitement ciblé du cuir chevelu qui est renforcé dans les zones de fragilisation du cuir chevelu. La configuration étoilée du support 32 permet à la fois un positionnement optimisé des diodes électroluminescentes 34 et permet d'avoir une flexibilité du support 32 facilitant une déformation curviligne pour se conformer à la courbure du casque 10.

Bien entendu, d'autres modes de réalisation sont envisageables sans sortir du cadre de l'invention. Ainsi, diverses modifications peuvent être apportées par l'homme du métier à l'invention qui vient d'être décrite à titre d'exemple.

## Revendications

1. Casque (10) de traitement du cuir chevelu d'un utilisateur par photothérapie, le casque (10) comprenant des parois de coque extérieure (22) et intérieure (24) s'étendant chacune d'une portion de sommet proximale à un bord distal et définissant une fois assemblées, une bordure périphérique (200) du casque (10) qui délimite une ouverture de passage de la tête de l'utilisateur, et une source de lumière (30) disposée entre les deux parois (22, 24) émettant vers l'intérieur du casque (10), la source de lumière (30) comprenant une pluralité d'émetteurs lumineux (34) et un support (32) d'interconnexion électrique d'émetteurs lumineux entre eux, **caractérisé en ce que** le support (32) est formé à partir d'une plaque ajourée qui est configurée en étoile et comprend une pluralité de branches principales (320) qui s'étendent radialement depuis une région centrale (R1) de convergence des branches principales (320) vers une extrémité libre des branches (320) à l'intérieur d'une région périphérique (R2) **et en ce que** au moins une branche principale (320) comprend une ramification en une pluralité de branches secondaires (330) partant d'au moins un des bords longitudinaux des branches principales (320).

2. Casque (10) selon la revendication précédente, dans lequel la densité de puissance dans la région centrale (R1) est maximale, de préférence 20% supérieure à la densité de puissance dans une région périphérique (R2) s'étendant autour de la région centrale (R1).

3. Casque (10) selon l'une quelconque des revendications précédentes, dans lequel la ramification des branches principales (320) diffère d'une branche principale (320) à l'autre.

4. Casque (10) selon l'une quelconque des revendications précédentes, dans lequel le support (32) est symétrique par rapport à un axe (X) s'étendant depuis une région frontale (RF) vers une région occipitale (RO) du casque (10).

5. Casque (10) selon l'une quelconque des revendications précédentes, dans lequel les émetteurs lumineux (34) sont plus concentrés dans la région centrale (R1) et plus dispersés en-dehors.

6. Casque (10) selon l'une quelconque des revendications précédentes, comprenant en outre une interface (26) de dissipation thermique métallique interposée entre les parois de coque extérieure (22) et intérieure (24) et supportant la source de lumière (30) du côté de sa face interne tournée vers la paroi de coque intérieure (24).

7. Casque (10) selon la revendication précédente, dans lequel l'interface de dissipation thermique (26) est formée par une plaque métallique conformée pour épouser la forme hémisphérique de la cavité du corps de casque (10).

8. Casque (10) selon la revendication précédente, dans lequel la plaque (26) est obtenue par pliage d'un flan métallique prédécoupé ou entaillé selon des lignes de pliage.

9. Casque (10) selon l'une quelconque des revendications précédentes, dans lequel la région centrale (R1) du support (32) s'étend au droit d'une région sommitale (RS) du casque (10).

10. Casque (10) selon la revendication précédente, dans lequel la région centrale (R1) du support (32) s'étend au droit d'une partie arrière ou d'une partie avant de la région sommitale (RS) du casque (10).

11. Casque (10) selon l'une quelconque des revendications précédentes, comprenant un bandeau circonférentiel (40) d'ajustement du casque (10) à un tour de tête de l'utilisateur, accroché à la bordure périphérique, ledit bandeau (40) comprenant un corps pourvu d'une portion de déformation s'étendant radialement vers l'intérieur du casque (10) et étant configurée pour se déformer entre une position de repos déployée radialement vers l'intérieur du casque (10) et une position tassée entre le casque (10) et la tête de l'utilisateur en configuration d'utilisation.
